# EUROPEAN PATENT APPLICATION

(11) **EP 4 603 081 A1**
(43) Date of publication of application: **20.08.2025**
(21) Application number: 25158485.0
(22) Date of filing: 18.02.2025
(51) Int. Cl.: A61K 9/00, A61K 9/08, A61K 31/4015, A61K 47/02, A61K 47/12

(54) **AN INJECTABLE COMPOSITION OF BRIVARACETAM**

(30) Priority: 19.02.2024 TR 202401925
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); ATAMAN, Seval, Istanbul (TR); YAZICI, Ozlem, Istanbul (TR)
(74) Representative: Genç Ilhan, Oznur

(57) **Abstract**

The present invention relates to an injectable composition comprising brivaracetam or a pharmaceutically acceptable salt thereof, a tonicity agent and at least one pH agent. Furthermore, a suitable process for this injectable composition is described.

## Description

### Field of the Invention

The present invention relates to an injectable composition comprising brivaracetam or a pharmaceutically acceptable salt thereof, a tonicity agent and at least one pH agent. Furthermore, a suitable process for this injectable composition is described.

### Background of the Invention

Epilepsy is a chronic disease in which brain neurons suddenly discharge abnormally, causing temporary brain dysfunction.

Brivaracetam is a commonly used auxiliary drug for the treatment of partial seizures in epilepsy. It is a selective and high-affinity ligand for synaptic vesicle protein.

The chemical name of brivaracetam is (2S)-2-[(4R)-2-oxo-4-propyl-pyrrolidin-1-yl]butanamide and its chemical structure is shown in the Formula I.

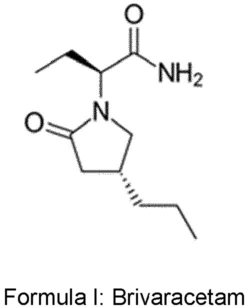

Brivaracetam is a third-generation antiepileptic racetam derivative and a 4-n-propyl analogue of levetiracetam. Brivaracetam empirical formula is C₁₁H₂0N₂O₂ and its molecular weight is 212.29.

Brivaracetam is a white to off-white crystalline powder. It is very soluble in water, buffer (pH 1.2, 4.5 and 7.4), ethanol, methanol, and glacial acetic acid. It is freely soluble in acetonitrile and acetone and soluble in toluene. It is very slightly soluble in n-hexane.

Brivaracetam is commercially approved in the form of tablets, oral solution and injection, in the USA under the brand name Briviact and are marketed by UCB Pharma. The product is indicated for the treatment of partial onset seizures only in adult patients. The commercially available Brivaracetam intravenous solution vial contains 50mg of brivaracetam and sodium acetate (trihydrate), glacial acetic acid, sodium chloride and water for injection as inactive ingredients. The USFDA product label for brivaracetam injection discloses it can be administered intravenously without further dilution or may be mixed with diluents like 0.9% Sodium Chloride injection USP, Lactated Ringer's injection, 5% Dextrose injection, USP. It also further discloses diluted solution should not be stored for more than 4 hours at room temperature and may be stored in polyvinyl chloride (PVC) bags.

Advantage of this invention exists in the fact that injection solution enables rapid intervention with respect to the patient who cannot implement achieve administration of the arbitrary formulations which pass oral ingestion in the case of an emergency or a crisis.

An injectable solution could be advantageously used in case of epilepsy crisis.

The patent CN113908120 disclose the invention relates to a medicine, and in particular to a brivaracetam medicine solution and a preparation method thereof. The brivaracetam medicine solution contains brivaracetam, a buffer pair and water.

The patent CN101945647 discloses the present invention concerns a stable pharmaceutical solution, a process of the preparation thereof and therapeutic uses thereof. The solution is sterilized by filtration through a conventional pathogen-impermeable filter, then dispensed into appropriate injectable containers and post-sterilized and obtained injectable forms.

There are many examples of injection formulations of brivaracetam in the prior art. Brivaracetam is unstable in aqueous solution and easily forms degradation products. Therefore, the stability of the drug solution for injection of Brivaracetam needs to be further improved. With this present invention, easy to use and stable pharmaceutical form will achieve.

We have tried new formulations in order to provide advantages over the prior art and we have seen that the use of sodium chloride as a tonicity agent in the injectable form of brivaracetam and the weight ratio of brivaracetam to sodium chloride is important.

### Detailed description of the Invention

The main object of the present invention is to provide a stable injectable composition of brivaracetam.

Another object of the present invention is to provide an injectable composition comprising brivaracetam having a long shelf life.

Solutions of brivaracetam for injection in the prior art was reported to be unstable. Therefore, a new formulation is still needed. We have found that an injectable composition with better stability and a prolonged shelf life is obtained when brivaracetam is used with sodium chloride and the weight ratio of brivaracetam to sodium chloride in the ratio stated below.

According to one embodiment of the invention, an injectable composition comprising brivaracetam or a pharmaceutically acceptable salt thereof, a tonicity agent and at least one pH agent, wherein the tonicity agent is sodium chloride and the weight ratio of brivaracetam to sodium chloride is between 0.5-1.5.

According to one embodiment of the invention, the weight ratio of brivaracetam to sodium chloride is between 0.8-1.3.

According to an embodiment of the present invention, the injectable composition comprising brivaracetam in an amount of 2 mg to 20 mg of 1 mL of the pharmaceutical composition.

According to an embodiment of the present invention, the injectable composition comprising sodium chloride in an amount of 1 mg to 20 mg of 1 mL of the pharmaceutical composition.

The effect of pH on brivaracetam stability is very important in developing the solution formulation of ionizable compounds, so the pH pairs chosen are very important.

Suitable pH agents are selected from the group comprising sodium bicarbonate, sodium carbonate, sodium citrate trihydrate, anhydrous citric acid, monosodium citrate, anhydrous disodium hydrogen phosphate, potassium carbonate, anhydrous sodium dihydrogen phosphate, dibasic potassium sulfate, nicotinic acid, dilute hydrochloric acid, glacial acetic acid, monobasic potassium phosphate, phosphoric acid, adipic acid, sodium acetate, sodium dihydrogen phosphate dihydrate, tribasic sodium phosphate or mixtures thereof.

In particular, we saw that stability was achieved when the following pH agent pairs were used.

According to an embodiment of the present invention, the pH agent is sodium bicarbonate, sodium carbonate, sodium citrate trihydrate, anhydrous citric acid or mixture thereof.

According to one embodiment of the invention, pH agents are sodium bicarbonate and sodium carbonate.

According to one embodiment of the invention, pH agents are sodium citrate trihydrate and anhydrous citric acid. We observed good stability with the use of these two pH agent combinations.

According to an embodiment of the present invention, the injectable composition comprising pH agents in an amount of 0.1 mg to 10 mg of 1 mL of the pharmaceutical composition.

Preferably, the pH of the injectable composition according to the present invention is 4.0 to 6.0. The pH is adjusted to these ranges with suitable pH agents to ensure stability throughout the shelf life of the product.

The invention injectable composition can be packaged in any form. In some embodiments, they are packaged in a syringe, vial, with or without a needle.

In one embodiment, the injectable composition comprises;
- brivaracetam
- sodium bicarbonate
- sodium carbonate
- sodium chloride.

In one embodiment, the injectable composition comprises;
- brivaracetam
- sodium citrate trihydrate
- anhydrous citric acid
- sodium chloride.

According to another embodiment of the present invention, a process for preparing the injectable composition comprising brivaracetam comprises the following steps;
a. Dissolving the required amount of sodium chloride in sterile water for injection,
b. Adding pH agent to the sodium chloride solution (0.9% w/v) obtained in step (a) and dissolving,
c. Adding Brivaracetam to the solution from step (b) and dissolving,
d. Adjusting the pH value of the solution obtained in step (c) to 5.5 using pH agent,
e. Adjust the volume using sodium chloride solution (0.9% w/v),
f. Filtering using PES filter.

### Example 1;

| **Ingredient** | **Amount in unit formula mg / mL** |
|---|---|
| Brivaracetam | 10,00 |
| Sodium bicarbonate | 1,64 |
| Sodium carbonate | qs |
| Sodium chloride | 9,00 |
| Purified water | qs to 1 ml |

| | |
|---|---|
| q.s.: quantity sufficient | |

1. Dissolving the required amount of sodium chloride in sterile water for injection and dissolving it completely,
2. Adding sodium bicarbonate to the sodium chloride solution (0.9% w/v) obtained in step 1 and dissolving it completely,
3. Adding Brivaracetam to the solution from step 2 and dissolving,
4. Adjusting the pH value of the solution obtained in step 3 to 5.5 using sodium carbonate,
5. Adjusting the volume to 5.00 ml using sodium chloride solution (0.9% w/v),
6. Filtering using a 0.2 micron PES filter.

### Example 2;

| **Ingredient** | **Amount in unit formula mg / mL** |
|---|---|
| Brivaracetam | 10,00 |
| Sodium citrate trihydrate | 1,64 |
| anhydrous citric acid | qs |
| Sodium chloride | 9,00 |
| Purified water | qs to 1 ml |

| | |
|---|---|
| q.s.: quantity sufficient | |

1. Dissolving the required amount of sodium chloride in sterile water for injection and dissolving it completely,
2. Adding sodium citrate trihydrate to the sodium chloride solution (0.9% w/v) obtained in step 1 and dissolving it completely,
3. Adding brivaracetam to the solution from step 2 and dissolving,
4. Adjusting the pH value of the solution obtained in step 3 to 5.5 using anhydrous citric acid ,
5. Adjusting the volume to 5.00 ml using sodium chloride solution (0.9% w/v),
6. Filtering using a 0.2 micron PES filter.

## Claims

1. An injectable composition comprising brivaracetam or a pharmaceutically acceptable salt thereof, a tonicity agent and at least one pH agent, wherein the tonicity agent is sodium chloride and the weight ratio of brivaracetam to sodium chloride is 0.5-1.5.

2. The injectable composition according to claim 1, wherein brivaracetam is present in an amount of 2 mg to 20 mg of 1 mL of the pharmaceutical composition.

3. The injectable composition according to claim 1, wherein sodium chloride is present in an amount of 1 mg to 20 mg of 1 mL of the pharmaceutical composition.

4. The injectable composition according to claim 1, wherein pH agents are selected from the group comprising sodium bicarbonate, sodium carbonate, sodium citrate trihydrate, anhydrous citric acid, monosodium citrate, anhydrous disodium hydrogen phosphate, potassium carbonate, anhydrous sodium dihydrogen phosphate, dibasic potassium sulfate, nicotinic acid, dilute hydrochloric acid, glacial acetic acid, monobasic potassium phosphate, phosphoric acid, adipic acid, sodium acetate, sodium dihydrogen phosphate dihydrate, tribasic sodium phosphate or mixtures thereof.

5. The injectable composition according to claim 4, wherein the pH agent is sodium bicarbonate, sodium carbonate, sodium citrate trihydrate, anhydrous citric acid or mixture thereof.

6. The injectable composition according to claim 4 or 5, wherein pH agents are sodium bicarbonate and sodium carbonate.

7. The injectable composition according to claim 4 or 5, wherein pH agents are sodium citrate trihydrate and anhydrous citric acid.
